# EUROPÄISCHE PATENTSCHRIFT

(11) **EP 1 731 120 B1**
(45) Veröffentlichungstag und Bekanntmachung des Hinweises auf die Patenterteilung: **07.05.2008**
(21) Anmeldenummer: 05405376.4
(22) Anmeldetag: 09.06.2005
(51) Int. Cl.: A61F 9/009

(54) **Ophthalmologische Vorrichtung für die Auflösung von Augengewebe**
Ophthalmological device for ablation of eye tissue.
Dispositif ophthalmologique destiné à l'ablation de tissus des yeux.

(43) Veröffentlichungstag der Anmeldung: 13.12.2006
(73) Patentinhaber: SIE AG, Surgical Instrument Engineering, 2562 Port (CH)
(72) Erfinder: Rathjen, Christian, 28197 Bremen (DE); Deyerler, Karl Michael, 85635 Höhenkirche-Siegertsbrunn (DE); Ripken, Tammo, 31515 Wunstorf (DE); Zesch, Wolfgang, 8047 Zürich (CH); Lubatschowski, Holger, 30989 Gehrden (DE)
(74) Vertreter: Vogel, Dany

(56) Entgegenhaltungen:
- EP-A- 1 486 185
- DE-A1- 3 724 282
- US-A1- 2004 243 112

## Beschreibung

### Technisches Gebiet

Die vorliegende Erfindung betrifft eine ophthalmologische Vorrichtung für die Auflösung von Augengewebe. Die Erfindung betrifft insbesondere eine ophthalmologische Vorrichtung, welche eine Basisstation mit einer Lichtquelle zur Erzeugung von Lichtpulsen sowie einen auf ein Auge aufsetzbaren Applikationskopf umfasst, welcher Applikationskopf versehen ist mit einem Lichtprojektor zur fokussierten Projektion der Lichtpulse für eine punktuelle Auflösung von Augengewebe und mit Bewegungstreibern zur Bewegung des Lichtprojektors in einer Vorschubrichtung und in einer ersten Abtastrichtung.

### Stand der Technik

Fehlsichtigkeiten wie Myopie (Kurzsichtigkeit), Hyperopie (Weitsichtigkeit oder Übersichtigkeit) oder Astigmatismus (Stabsichtigkeit) können heute durch refraktiv-chirurgische Behandlung dauerhaft korrigiert werden. Refraktiv-chirurgische Behandlungen sind chirurgische Eingriffe am Auge, die die optische Brechkraft des Auges ändern mit dem Ziel, diese einem gewünschten Wert möglichst gut anzunähern. Eines der bedeutendsten Verfahren in der refraktiven Chirurgie ist die so genannte Laser in-situ Keratomileusis (LASIK), bei der das Innere der Hornhaut mit einem computergesteuerten Eximerlaser abgetragen wird, nachdem zuvor ein Hornhautlappen teilweise abgetrennt und weggeklappt wird. Zur Erzeugung des Hautlappens werden mechanische Mikrokeratome eingesetzt, bei denen ein angetriebenes Messer den Hautlappen schneidet. Neuerdings können solche Hautlappen auch mit stark fokussierten Femtosekundenlaserpulsen geschnitten werden, die Pulsbreiten von typisch 100fs bis 1000fs (1fs=10⁻¹⁵s) aufweisen. Die beim Einsatz eines mechanisch oszillierenden Messers bestehenden Gefahren werden bei der Verwendung eines Femtosekundenlasers vermieden. Ein solches System wird z. B. von der Firma IntraLase Corp, in Irvine, California, USA, unter dem Namen Pulsion FS Laser angeboten. Die Baugrösse der bekannten Systeme mit Femtosekundenlaser ist mit der Baugrösse eines Eximerlasers vergleichbar, was den Nachteil hat, dass im Behandlungsraum der Platz, der für den Eximerlaser benötigt wird, ein weiteres Mal für das Femtosekundenlasersystem benötigt wird. Zudem muss der Patient nach dem Schneiden des Hautlappens vom Femtosekundenlasersystem zum Eximerlaser transferiert werden. Die Baugrösse der Femtosekundenlaser wird unter anderem durch die verwendete Lichtquelle, die Scannertechnologie und die damit einhergehenden Strahlführungssysteme bestimmt.

Innerhalb eines grossen stationären Linsensystems wird der Laserstrahl durch strahlumlenkende optische Elemente auf die zu trennenden Gewebebereiche des Auges fokussiert. Konstruktionsbedingt ist dabei die maximal erreichbare numerische Apertur (NA proportional zum halben Sinus des Öffnungswinkel des Objektivs) der Fokussieroptik begrenzt (typisch NA=0.2-0.3). Bei gegebenem Arbeitsfeld, beispielsweise die gesamte Hornhaut, erfordert die Scanoptik (typischerweise eine f-Theta Optik) einen Mindestarbeitsabstand. Aus dem Arbeitsabstand ergibt sich im Zusammenhang mit der erforderlichen Bewegung und der erreichbaren Grösse der strahlumlenkenden optischen Elemente eine konstruktive Grenze für den Durchmesser der Scanoptik. Eine weitere konstruktive Obergrenze für den Durchmesser ergibt sich durch Abschattungen beziehungsweise Kollisionen mit Körperteilen (Augenbrauen, Nase). Auch bei grossen Durchmessern kann immer nur ein Teilbereich der Optik vom scannenden Laserstrahl ausgeleuchtet werden. Damit ergibt sich eine konstruktive Obergrenze für die effektive ausnutzbare numerische Apertur der Optik. Hohe Aperturen sind wünschenswert, weil sich mit hohen NA kleine Brennpunkte und damit eine kleinere Schnittzone pro Puls erzeugen lassen. In kleineren Schnittzonen wird pro Puls weniger Gas erzeugt, als in grossen Schnittzonen. Durch kleinere Gasblasen lassen sich präzisere Schnitte erzeugen, da unter anderem die Schnittzonen nicht wesentlich durch den internen Gasdruck deformiert werden. Zudem benötigen hohe NA überproportional weniger Energie pro Puls zur Erzeugung eines Schnittes. Mit geringerer Energie reduzieren sich auch die durch den Laserpuls erzeugten Kavationsblasen, was sich zusätzlich positiv auf die Schnittqualität auswirkt. Weiterhin wird die Netzhaut durch die stärker divergierenden Strahlen hinter dem Brennpunkt bei hoher NA weniger belastet. Ein weiterer Vorteil ist, dass bei hoher NA lokale Verschmutzungen in der Nähe der Hornhautoberfläche die Intensität im Fokus weniger reduzieren.

In der Patentschrift US 5,549,632 wird eine ophthalmologische Vorrichtung mit einer Laserquelle für die Auflösung von Augengewebe beschrieben, welche unter anderem für das Schneiden von Hornhautlappen einsetzbar ist. Die Vorrichtung gemäss US 5,549,632 umfasst eine Laserquelle sowie einen mit der Laserquelle optisch verbundenen Projektionskopf in einem von der Laserquelle separaten Gehäuse. Die Vorrichtung gemäss US 5,549,632 umfasst zudem Strahlsteuermittel, die den Strahlengang der von der Laserquelle abgegebenen Laserpulse so steuern, dass Punkte in einem zur Laserquelle festen Referenzrahmen über eine optische Verbindung auf entsprechende Punkte in einem zum Projektionskopf festen Referenzrahmen abgebildet werden. Damit die durch die Strahlsteuermittel abgelenkten Lichtpulse relativ zum Referenzrahmen des Handgeräts abgebildet werden können, ist die optische Verbindung als Spiegelgelenkarm ausgeführt. Durch die Verbindung des Projektionskopfs mit einer fest mit dem Auge verbindbaren Applanationsplatte wird der feste Referenzrahmen des Projektionskopfs gemäss US 5,549,632 fest auf die Applanationsplatte und somit auf das Auge abgebildet. Gemäss US 5,549,632 werden die Laserpulse an gewünschte Positionen des Auges geleitet, indem die Strahlsteuermittel die Position des gepulsten Laserstrahls relativ zu der Applanationsplatte steuern und über die optische Verbindung und das optische Projektionssystem des Projektionskopfs auf das Auge abbilden. Um beispielsweise Schnitte von 5 bis 15 mm Länge durchzuführen muss das optische Projektionssystem des Projektionskopfs optische Linsen aufweisen, deren Durchmesser grösser als der Durchmesser des Augapfels ist. Durch einen derart gross dimensionierten Projektionskopf wird die Sicht auf das zu behandelnde Auge verdeckt. Weiterhin ist die numerische Apertur der Vorrichtung gemäss US 5,549,632 gering, wie aus der geringeren Konvergenz der Strahlen ersichtlich ist. Grosse Linsensysteme haben zudem den Nachteil, dass durch sie Vorrichtungen schwer und unhandlich werden, wodurch manuelles Halten und Applizieren erschwert wird.

In der Patentanmeldung EP 1 486 185 wird eine ophthalmologische Vorrichtung für die Auflösung von Augengewebe beschrieben, welche einen auf das Auge aufsetzbaren Applikationskopf umfasst, welcher mit einem Lichtprojektor versehen ist. Der Lichtprojektor wird mittels Bewegungstreibern bewegt, um den Brennpunkt für die Gewebeauflösung an die gewünschten Stelle zu bringen. Nach EP 1 486 185 kann den translatorischen Bewegungen des Lichtprojektors mittels optischer Microscans zusätzlich eine feine Bewegung des Brennpunkts überlagert werden. Um jedoch für das Schneiden vorteilhafte, gross aufgeweitete, nicht konvergente Lichtstrahlen ablenken zu können, sind relativ grosse Spiegel (z. B. 14mm) erforderlich, die um relativ grosse Winkel (z. B. 4 Grad) verkippt werden müssen. Derart grosse Scannersysteme lassen jedoch mit dem gegenwärtigen Stand der Technik keine grossen Geschwindigkeiten zu und können nicht kompakt gebaut werden. Wenn die oben genannten Nachteile einer grossen Baugrösse des Applikationskopfs vermieden werden sollen und wenn verhindert werden soll, dass die gleiche Stelle im Augengewebe vielfach von den Lichtpulsen getroffen wird, können im Applikationskopf allerdings bloss optische Microscans für kleine Pulsraten eingesetzt werden. Kleine Pulsraten bedeuten allerdings einen verlangsamten und damit weniger stabilen Operationsvorgang.

### Darstellung der Erfindung

Es ist eine Aufgabe der vorliegenden Erfindung, eine neue ophthalmologische Vorrichtung für die Auflösung von Augengewebe vorzuschlagen, welche mindestens gewisse Nachteile des Stands der Technik nicht aufweist.

Gemäss der vorliegenden Erfindung werden diese Ziele insbesondere durch die Elemente der unabhängigen Ansprüche erreicht. Weitere vorteilhafte Ausführungsformen gehen ausserdem aus den abhängigen Ansprüchen und der Beschreibung hervor.

Die ophthalmologische Vorrichtung umfasst eine Basisstation mit einer Lichtquelle zur Erzeugung von Lichtpulsen sowie einen auf ein Auge aufsetzbaren Applikationskopf. Der Applikationskopf ist mit einem Lichtprojektor zur fokussierten Projektion der Lichtpulse für eine punktuelle Auflösung von Augengewebe und mit Bewegungstreibern zur Bewegung des Lichtprojektors in einer Vorschubrichtung und in einer ersten Abtastrichtung versehen. Der Applikationskopf umfasst beispielsweise einen auf das Auge aufsetzbaren, mindestens stellenweise lichtdurchlässigen Kontaktkörper, welcher so ausgebildet und angeordnet ist, dass er einen im aufgesetzten Zustand kontaktierten Bereich des Auges äquidistant zu einer Arbeitsfläche setzt. Der Applikationskopf weist beispielsweise auch Befestigungsmittel auf zum Fixieren des Applikationskopfs durch Unterdruck am Auge.

Die oben genannten Ziele werden durch die vorliegende Erfindung insbesondere dadurch erreicht, dass die ophthalmologische Vorrichtung in der Basisstation angeordnete Strahlablenkungsmittel umfassen zur Ablenkung der Lichtpulse in einer zweiten Abtastrichtung, beispielsweise einen drehbar gelagerten Spiegel mit Antriebsmitteln, und ein optisches Übertragungssystem aufweist zur Übertragung abgelenkter Lichtpulse von der Basisstation zum Applikationskopf und zur Überlagerung der in der zweiten Abtastrichtung abgelenkten Lichtpulse auf die Bewegung des Lichtprojektors in der ersten Abtastrichtung. Der drehbar gelagerte Spiegel ist beispielsweise als Polygonspiegel mit mehreren Facetten ausgestaltet. Die Strahlablenkungsmittel umfassen in einer alternativen Variante AOM-Elemente (akustisch/optische Modulatoren). Die Bewegung des Lichtprojektors ("Slowscan") in der ersten Abtastrichtung ist vorzugsweise im Vergleich zu einer durch die Ablenkung der Lichtpulse in der zweiten Abtastrichtung verursachten Bewegung der Lichtpulse ("Fastscan") um ein Vielfaches langsamer. Die Auslagerung des den drehbar gelagerten Spiegel und die Antriebsmittel umfassenden Scanners in die Basisstation und die optische Übertragung und Überlagerung der mittels des Scanners in der zweiten Abtastrichtung abgelenkten Lichtpulse auf die Slowscan-Bewegung des Lichtprojektors im Applikationskopf ermöglicht eine wesentlich kleinere Baugrösse des Applikationskopfs als eine Konfiguration des Applikationskopfs mit einem Scanner für Fastscan-Bewegungen. Es können Lichtquellen mit hohen Lichtpulsraten, beispielsweise Femtosekundenlaser, verwendet werden, ohne dass die Baugrösse des Applikationskopfs durch einen Scanner für Fastscan-Bewegungen unpraktisch vergrössert werden muss. Die Baugrösse des Applikationskopfs kann so dimensioniert werden, dass der Applikationskopf im Behandlungsraum an einem Patienten appliziert werden kann, der unter einem Eximerlasersystem positioniert ist, das heisst der Applikationskopf kann zwischen Eximerlasersystem und Patient eingeschoben werden, so dass während der gesamten Behandlung weder der Patient noch das Eximerlasersystem verschoben und neu positioniert werden müssen. Mittels des ausgelagerten Scanners für Fastscan-Bewegungen kann verhindert werden, dass bei Verwendung von Lichtquellen mit hohen Lichtpulsraten die gleiche Stelle im Augengewebe vielfach von den Lichtpulsen getroffen wird. Durch die Verwendung von Lichtquellen mit hohen Lichtpulsraten kann der Operationsvorgang schneller und damit stabiler durchgeführt werden als bei der Verwendung von Lichtquellen mit niedrigeren Lichtpulsraten, was die Genauigkeit der Behandlung erhöht und die Belastung des Auges reduziert.

In einer bevorzugten Ausführungsvariante ist an der Basisstation ein Gelenkarm angebracht, welcher mit einem von der Basisstation abgewandten Ende mit dem Applikationskopf befestigbar ist, und das optische Übertragungssystem ist im Innern des Gelenkarms angeordnet. Der Gelenkarm ermöglicht eine flexible Positionierung des Applikationskopfs bei gleichzeitiger Übertragung des Gewichts des Applikationskopfs auf die Basisstation und daraus resultierender Entlastung des zu behandelnden Auges.

Vorzugsweise umfasst der Gelenkarm mehrere Armelemente, wobei jeweils zwei der Armelemente mittels eines Gelenkmoduls so verbunden sind, dass sie in parallelen Ebenen zueinander verdrehbar sind, wobei mindestens eines der Armelemente um seine Längsachse drehbar ist, und wobei zur Übertragung der abgelenkten Lichtpulse von einem Armelement zum anderen Armelement in den Gelenkmodulen Umlenkspiegel angebracht sind.

In einer Ausführungsvariante sind an mindestens einem beweglichen Armelement des Gelenkarms Mittel zur Gewichtskompensation angebracht, um den Applikationskopf im Gleichgewicht zu halten, beispielsweise Gegengewichte, Federn oder Aktoren.

In einer bevorzugten Ausführungsvariante umfasst das optische Übertragungssystem ein Rotationselement, das beispielsweise als K-Spiegel ausgestaltet ist, zum Drehen einer durch die abgelenkten Lichtpulse definerten Abtastebene um eine optische Übertragungsachse. Das Rotationselement ist vorzugsweise in der Basisstation angeordnet. Das Rotationselement ermöglicht Bilddrehungen, die durch den Spiegelgelenkarm verursacht werden, auf einfache Weise zuverlässig zu kompensieren.

In einer Ausführungsvariante umfasst der Applikationskopf einen Sender zum Aussenden einer Lichtmarkierung zur Bestimmen einer Sollausrichtung der zweiten Abtastrichtung. Die Lichtmarkierung ist so angeordnet, dass sie über das optische Übertragungssystem zu der Basisstation übertragbar ist. Zudem umfasst die Basisstation ein Lichtsensormodul und ein mit dem Lichtsensormodul verbundenes Regelmodul. Das Regelmodul ist eingerichtet das Rotationselement so zu steuern, dass die durch die abgelenkten Lichtpulse definierte Abtastebene so lange um die optische Übertragungsachse gedreht wird, bis die Sollausrichtung der zweiten Abtastrichtung erreicht worden ist.

In einer Ausführungsvariante umfasst das Lichtsensormodul zwei Lichtsensoren zum Detektieren der über das optische Übertragungssystem zu der Basisstation übertragenen Lichtmarkierung und das Regelmodul ist eingerichtet das Rotationselement auf Grund einer Summe und/oder einer Differenz von durch die Lichtsensoren bestimmten Lichtstärken der detektierten Lichtmarkierung zu steuern.

In einer alternativen Ausführungsvariante sind mindestens gewisse der Armelemente mit einem Rotationssensor versehen, welche zur Rückmeldung eines Verdrehungsgrads des betreffenden Armelements mit einem Regelmodul verbunden sind. Das Regelmodul ist in dieser alternativen Ausführungsvariante eingerichtet, das Rotationselement so zu steuern, dass die durch die abgelenkten Lichtpulse definierte Abtastebene so lange um die optische Übertragungsachse gedreht wird, bis eine Sollausrichtung der zweiten Abtastrichtung erreicht worden ist.

In einer bevorzugten Ausführungsvariante umfasst die Basisstation ein Steuermodul, welches eingerichtet ist die Bewegungstreiber so zu steuern, dass der Lichtprojektor äquidistant eine Arbeitsfläche (Schnittfläche) in Zeilen, die in der ersten Abtastrichtung verlaufen, mit einem definierten Zeilenvorschub in der Vorschubrichtung abfährt. Dabei sind das optische Übertragungssystem und der Lichtprojektor optisch so gekoppelt, dass die in der zweiten Abtastrichtung abgelenkten Lichtpulse den Zeilen überlagert werden, so dass die punktuelle Auflösung von Augengewebe in der Arbeitsfläche zusammenhängend erfolgt.

In einer Ausführungsvariante sind die Lichtquelle und der Lichtprojektor eingerichtet für die punktuelle Auflösung von Augengewebe an einem Brennpunkt im Innern des Auges. Die Lichtquelle umfasst insbesondere einen Femtosekundenlaser. Ein Femtosekundenlaser umfasst einen Lasergenerator zur Erzeugung von Laserpulsen mit Pulsbreiten von 1fs bis 1ps (1fs=1/1000ps=10⁻¹⁵s). Laserpulse mit solchen Pulsbreiten ermöglichen die gezielte punktuelle Auflösung von Gewebe im Innern des Augengewebes, wobei mechanische und thermische Nebeneffekte im umgebenden Gewebe, wie man sie von längeren Pulsbreiten kennt, stark reduziert werden. Zudem umfasst die Basisstation ein Steuermodul, welches eingerichtet ist die Bewegungstreiber so zu steuern, dass durch die Lichtpulse, die in der zweiten Abtastrichtung abgelenkt und der Bewegung des Lichtprojektors in der ersten Abtastrichtung überlagert sind, ein Gewebelappen, der in einem Restbereich mit dem Auge verbunden bleibt, vom verbleibenden Augengewebe getrennt wird.

### Kurze Beschreibung der Zeichnungen

Nachfolgend wird eine Ausführung der vorliegenden Erfindung anhand eines Beispieles beschrieben. Das Beispiel der Ausführung wird durch die folgenden beigelegten Figuren illustriert:
Figur 1 zeigt ein Blockdiagramm, welches schematisch eine ophthalmologische Vorrichtung darstellt, die einen Applikationskopf aufweist, welcher über einen Gelenkarm mit einer Basisstation verbunden ist.
Figur 2 zeigt einen schematischen Querschnitt des Gelenkarms mit einem darin angebrachten optischen Übertragungssystem.
Figur 3 zeigt einen schematischen Querschnitt des Applikationskopfs, welcher mittels einer Saugeinheit an einem Auge angebracht ist.
Figur 4 zeigt ein Blockdiagramm, welches schematisch die zusammenwirkenden funktionalen Elemente der ophthalmologischen Vorrichtung darstellt.
Figur 5 zeigt eine Aufsicht auf ein Auge, von welchem mittels einer auf eine mechanische Bewegung überlagerten schnellen Scanbewegung eines Laserstrahls ein Gewebelappen getrennt wurde, der in einem Restbereich mit dem Auge verbunden bleibt.
Figur 6 illustriert die Überlagerung einer schnellen Scanbewegung eines gepulsten Laserstrahls auf eine langsamere mechanische Bewegung des Laserstrahls.
Figur 7 zeigt ein Blockdiagramm, welches schematisch die Übertragung einer Lichtmarkierung als Positionierungshilfe von einer Senderebene über das optische Übertragungssystem des Gelenkarms und über ein Rotationselement zu einer Empfängerebene illustriert.
Figur 8 zeigt den Verlauf eines Summensignals und eines Differenzsignals, welche in der Empfängerebene basierend auf der empfangenen Lichtmarkierung gebildet werden.

### Wege zur Ausführung der Erfindung

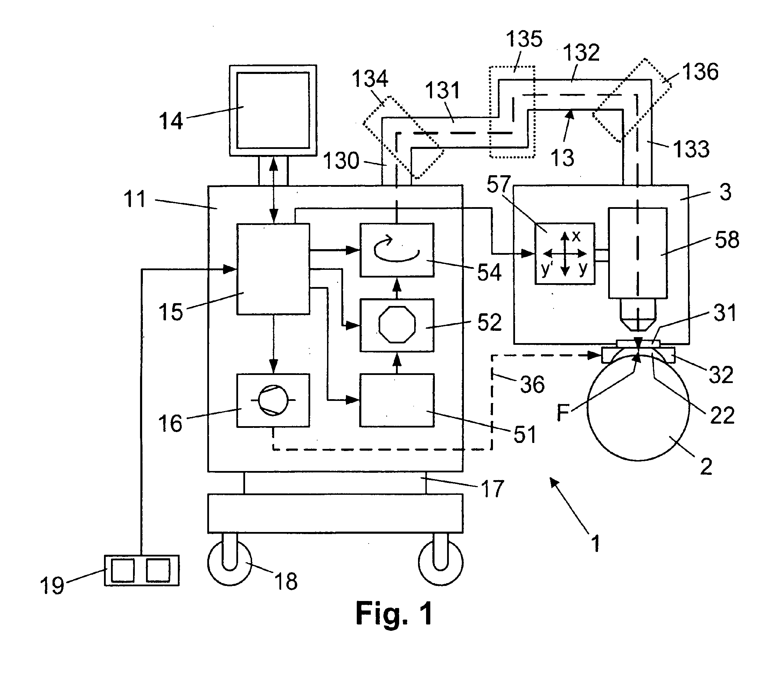

In den Figuren 1 bis 8 werden einander entsprechende Komponenten durch gleiche Bezugszeichen bezeichnet.

In der Figur 1 bezieht sich das Bezugszeichen 1 auf eine ophthalmologische Vorrichtung, welche eine Basisstation 11 und einen Applikationskopf 3 umfasst, die über einen Gelenkarm 13 miteinander verbunden sind.

Die Basisstation 11 ist mobil, ausgestaltet und verfügt über ein arretierbares Fahrwerk 18. Zudem verfügt die Basisstation 11 über eine Höhenverstellung 17, welche ermöglicht die Höhe der Basisstation 11 zu justieren. Die Basisstation 11 umfasst zudem ein elektronisches Steuermodul 15, welches eingerichtet ist, Steuersignale von einem Benutzer über einen zum Gehäuse der Basisstation 11 extern angeordneten Fussschalter 19 entgegenzunehmen. Das Steuermodul 15 ist zudem von einem Benutzer über eine Anzeige- und Bedieneinheit 14 bedien- und steuerbar. Die Basisstation 11 verfügt weiter über eine Vakuumpumpe 16, eine Lichtquelle 51, einen Scanner 52 und ein Rotationselement 54, die durch das Steuermodul 15 gesteuert werden. Das Steuermodul 15 steuert zudem die Bewegungstreiber 57 des Applikationskopfs 3.

Die Vakuumpumpe 16 ist über eine Vakuumanschlussleitung 36 mit einer Ansaugeinheit 32 des Applikationskopfs 3 verbunden. Über die Ansaugeinheit 32, die beispielsweise in der Form eines Ansaugrings ausgestaltet ist, ist der Applikationskopf 3 am zu behandelnden Auge 2 fixierbar. Beim Fixieren des Applikationskopfs 3 am Auge 2 wird die Oberfläche des zu behandelnden Bereichs des Auges 2 durch einen Kontaktkörper 31 des Applikationskopfs 3 applaniert, oder, abhängig von der Formgestaltung des Kontaktkörpers 31, in eine andere gewünschte Form gebracht, z.B. konkav oder konvex. Der Kontaktkörper 31 dient somit als Referenzkörper, der das zu bearbeitende Augengewebe 22 in eine definierte Lage zum Applikationskopf 3 bringt.

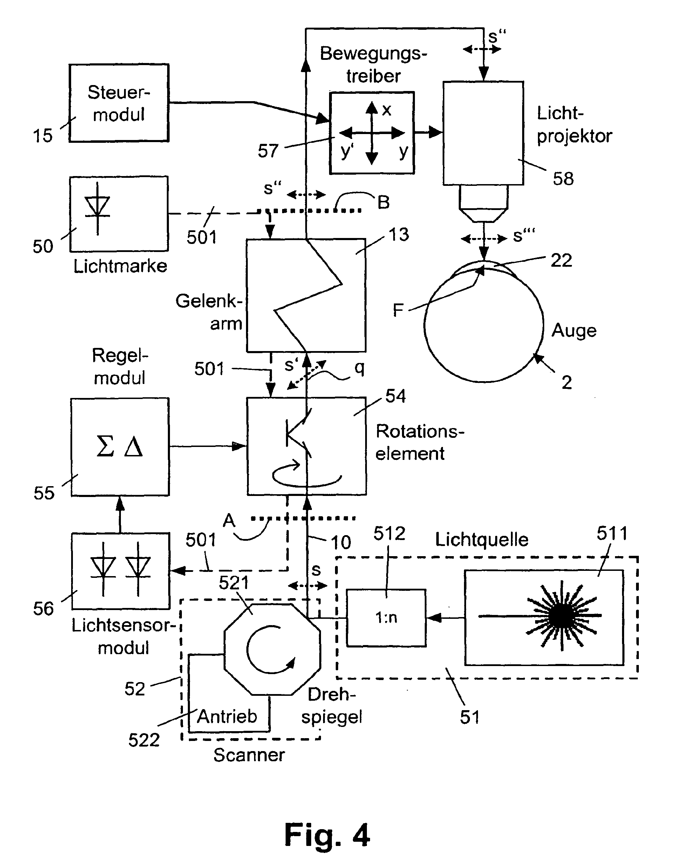

Wie in der Figur 4 schematisch dargestellt ist, umfasst die Lichtquelle 51 einen Femtosekundenlaser 511, das heisst einen Lasergenerator zur Erzeugung von Laserpulsen mit Pulsbreiten im Femtosekundenbereich von 1fs bis 1ps (1fs=1/1000ps=10⁻¹⁵s). Die Lichtquelle 51 umfasst zudem einen mit dem Femtosekundenlaser 511 verbundenen Strahlaufweiter 512, der dazu dient, den Strahldurchmesser für die Übertragung über das nachfolgende optische Übertragungssystem aufzuweiten und damit die Divergenz zu verkleinern. Strahlaufweitungen/Reduzierungen hinter dem Spiegel erlauben darüber hinaus eine Anpassung des Scanwinkels.

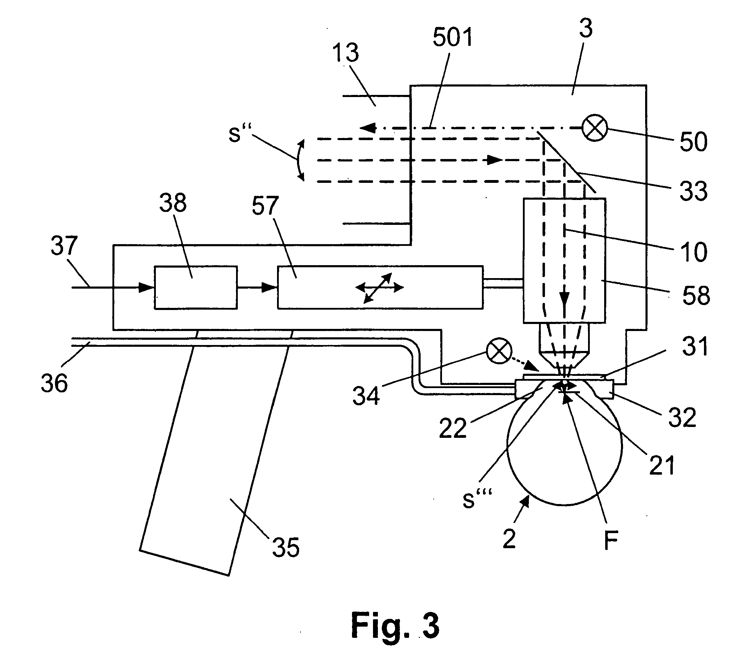

Die Bewegungstreiber 57 umfassen vorzugsweise ein Antriebselement für eine Vorschubrichtung x und ein Antriebselement für eine Abtastrichtung y, y', beispielsweise Piezomotoren. Der Applikationskopf 3 umfasst zudem einen Lichtprojektor 58, der optisch mit der Lichtquelle 51 verbunden ist, wie später näher ausgeführt wird, und der als Schneidobjektiv zur fokussierten Projektion von Lichtpulsen für die punktuelle Gewebeauflösung an einem Brennpunkt F im Innern des Augengewebes 22 eingerichtet ist. Wie aus der Figur 3 ersichtlich ist, sind die Bewegungstreiber 57 eingerichtet und angeordnet, den Lichtprojektor 58 mechanisch äquidistant (vorzugsweise parallel) zu einer Schnittfläche 21 (Arbeitsfläche) zu bewegen, so dass der Brennpunkt F in der Schnittfläche 21 verschoben wird. In einer Ausführungsvariante umfassen die Bewegungstreiber 57 überdies ein Antriebselement, um den Lichtprojektor 58 für eine Tiefeneinstellung des Brennpunkts F senkrecht zu einer durch die Vorschubrichtung x und Abtastrichtung y, y' aufgespannten Ebene zu bewegen.

Der Applikationskopf 3 ist über ein Verbindungsstück 133 mit dem Gelenkarm 13 entfernbar verbunden, beispielsweise mittels einer Schraubverbindung oder einem Schnappverschluss. Am vom Applikationskopf 3 abgewandten Ende des Gelenkarms 13, ist der Gelenkarm 13 über ein Anschlussstück 130 an der Basisstation 11 angebracht. Der Gelenkarm 13 weist ein als Schultergelenk 134 bezeichnetes Gelenkmodul auf, welches ein als Oberarm 131 bezeichnetes Armelement beweglich mit dem Anschlussstück 130 verbindet. Der Gelenkarm 13 weist ein als Handgelenk 136 bezeichnetes Gelenkmodul auf, welches ein als Unterarm 131 bezeichnetes Armelement beweglich mit dem Verbindungsstück 133 verbindet. Der Gelenkarm 13 weist zudem ein als Ellbogengelenk 135 bezeichnetes Gelenkmodul auf, welches den Oberarm 131 beweglich mit dem Unterarm 132 verbindet.

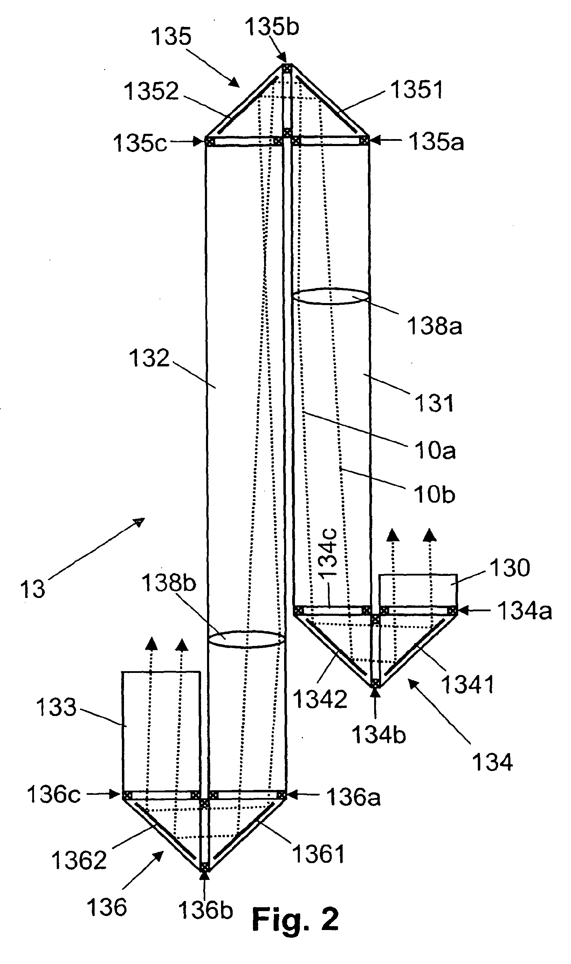

Wie in der Figur 2 schematisch dargestellt ist, umfassen das Schultergelenk 134, das Ellbogengelenk 135 sowie das Handgelenk 136 jeweils ein Drehgelenk 134b, 135b respektive 136b, welches ermöglicht die am betreffenden Gelenkmodul angebrachten Armelemente in parallelen Ebenen zueinander zu verdrehen. Überdies umfassen das Schultergelenk 134, das Ellbogengelenk 135 sowie das Handgelenk 136 jeweils weitere Drehgelenke 134a und 134c, 135a und 135c respektive 136a und 136c, welche ermöglichen die am betreffenden Gelenkmodul angebrachten Armelemente um ihre Längsachsen zu verdrehen, wobei es auch möglich ist eines der Gelenke 134c/135a bzw. 135c/136a zu eliminieren. Der Gelenkarm umfasst zudem ein optisches Übertragungssystem, welches Linsensysteme 138a, 138b und Umlenkspiegel 1341, 1342, 1351, 1352, 1361, 1362 umfasst. Die Umlenkspiegel 1341, 1342, 1351, 1352, 1361, 1362 sind paarweise in einem der Gelenkmodule angeordnet, wobei jeder der Umlenkspiegel 1341, 1342, 1351, 1352, 1361, 1362 mit einem 45° Winkel zur Längsachse des direkt mit dem betreffenden Gelenkmodul verbundenen Armelements angeordnet ist und wobei jeweils das in einem der Gelenkmodule angeordnete Paar der Umlenkspiegel 1341, 1342, 1351, 1352, 1361, 1362 in der in der Figur 2 dargestellten, zusammengefalteten Position senkrecht zueinander angeordnet ist. Wie mittels der beispielhaften Strahlen 10a, 10b schematisch dargestellt wird, ermöglichen die Linsensysteme 138a, 138b und die Umlenkspiegel 1341, 1342, 1351, 1352, 1361, 1362 einen bidirektionalen Strahlengang von einem Ende des Gelenkarms 13 zum anderen Ende des Gelenkarms 13, so dass Lichtstrahlen in sämtlichen möglichen Ausrichtungen des Gelenkarms 13 sowohl von der Basisstation 11 zum Applikationskopf 3 als auch vom Applikationskopf 3 zur Basisstation 11 übertragen werden können. Die Bidirektionalität des optischen Übertragungssystems des Gelenkarms 13 ist insbesondere im Zusammenhang mit der Übertragung einer Referenzlichtmarke vom Applikationskopf 3 zur Basisstation 11 wichtig, was später detaillierter beschrieben wird.

Wie in der Figur 3 dargestellt ist, weist der Applikationskopf 3 einen Handgriff 35 zur manuellen Positionierung des Applikationskopfs 3 auf. Zudem umfasst der Applikationskopf 3 ein Elektronikmodul 38, das über eine Verbindungsschnittstelle 37 mit dem Steuermodul 15 der Basisstation 11 verbunden ist und die Bewegungstreiber 57 ansteuert. Der Applikationskopf 3 verfügt auch über eine Augenbeleuchtung 34 zur Projektion von Referenzmarkierungen auf das Auge 2, welche als Positionierungshilfen dienen. Der Applikationskopf 3 ist mit einem Sichtfenster versehen, um die Referenzmarkierung für den Benutzer beim Aufsetzen des Applikationskopfs 3 auf das Auge 2 sichtbar zu machen. Der Applikationskopf 3 umfasst zudem einen Sender 50 zum Aussenden einer Lichtmarkierung, beispielsweise eine LED (Light Emitting Diode). Der Sender 50 erzeugt die Lichtmarkierung in Form eines Referenzlichtstrahls 501, der vom Applikationskopf 3 über das optische Übertragungssystem des Gelenkarms 13 zur Basisstation 11 übermittelt wird. Wie aus der Figur 3 ersichtlich ist, umfasst der Applikationskopf 3 einen Umlenkspiegel 33, der so angeordnet ist, dass ein Laserstrahl 10, der über das optische Übertragungssystem des Gelenkarms 13 von der Basisstation 11 empfangen wird, zum Lichtprojektor 58 umgelenkt wird, so dass eine schnelle Scanbewegung des Laserstrahls 10 in der Abtastrichtung s" die mechanische Bewegung des Lichtprojektors 58, welche durch die Bewegungstreiber 57 erzeugt wird, überlagert. Dadurch wird eine kombinierte Scanbewegung s''' des Brennpunkts F erzeugt, welche auf der schnellen Scanbewegung in der Abtastrichtung s" des Laserstrahls 10 und einer langsamen mechanischen Bewegung des Lichtprojektors 58 in der Vorschubrichtung x und in der Abtastrichtung y, y' basiert und im Augengewebe 22 die Schnittfläche 21 erzeugt.

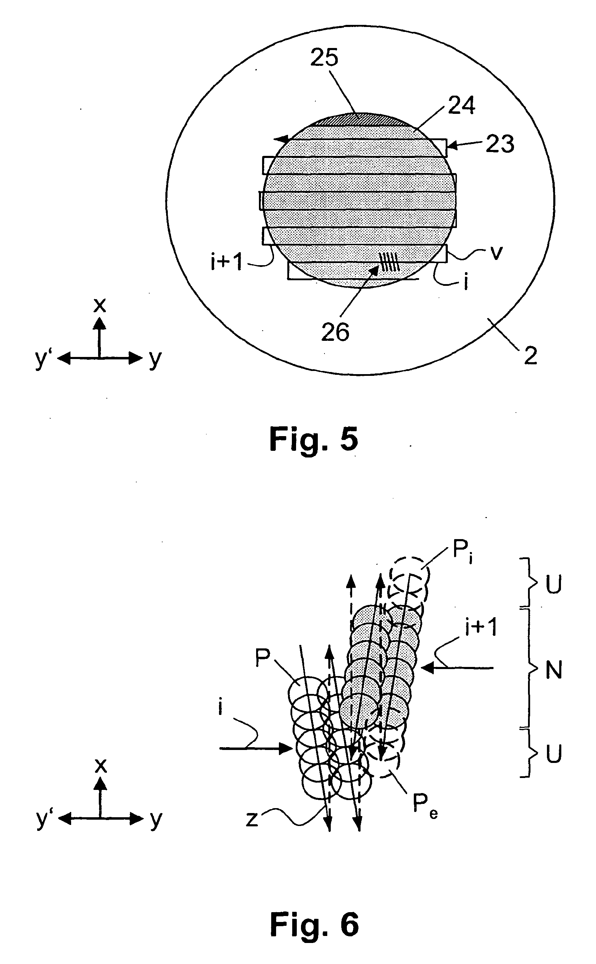

Wie in der Figur 4 schematisch dargestellt ist, werden die schnellen Scanbewegungen des Laserstrahls 10 durch den Scanner 52 erzeugt. Der Scanner 52 weist einen drehbar gelagerten Spiegel 521 sowie Antriebsmittel 522 auf, welche den drehbaren Spiegel 521 um eine Rotationsachse rotieren. Die von der Lichtquelle 51 abgegebenen Lichtpulse werden vom rotierenden Spiegel 521 in der Abtastrichtung s abgelenkt. Der Spiegel 521 ist vorzugsweise ein Polygonspiegel mit mehreren Facetten, beispielsweise ein achteckiger Pyramidenspiegel. Die Lichtpulse werden durch jede Facette erneut, wie in der Figur 6 dargestellt, ausgehend von einer Ausgangsposition Pᵢ bis zu einer Endposition Pₑ abgelenkt. Nachdem der durch die Lichtpulse gebildete Lichtstrahl auf das Ende der Facette des rotierenden Spiegels 521 trifft, wird der Lichtstrahl von der nächsten Facette wiederum von der Ausgangsposition Pᵢ bis zur Endposition Pₑ abgelenkt. Der Laserstrahl 10, der durch die in der Abtastrichtung s abgelenkten Lichtpulse gebildet wird, wird über das Rotationselement 54, den verspiegelten Gelenkarm 13 sowie den Umlenkspiegel 33 auf den Lichtprojektor 58 des Applikationskopfs 3 übertragen, wie nachfolgend näher beschrieben wird. Wie in der Figur 6 dargestellt ist, ergibt sich durch die Überlagerung der abgelenkten Lichtpulse auf die mechanische Bewegung des Lichtprojektors 58 eine Projektion von Lichtpulsen, die einer Sägenzahnkurve in der Abtastrichtung y respektive y' der mechanischen Bewegung des Lichtprojektors 58 folgt. In einer Ausführungsvariante umfasst die Basisstation 11 einen weiteren Scanner um die von der Lichtquelle 51 abgegebenen Lichtpulse mittels eines weiteren rotierenden Spiegels senkrecht zur Abtastrichtung s abzulenken.

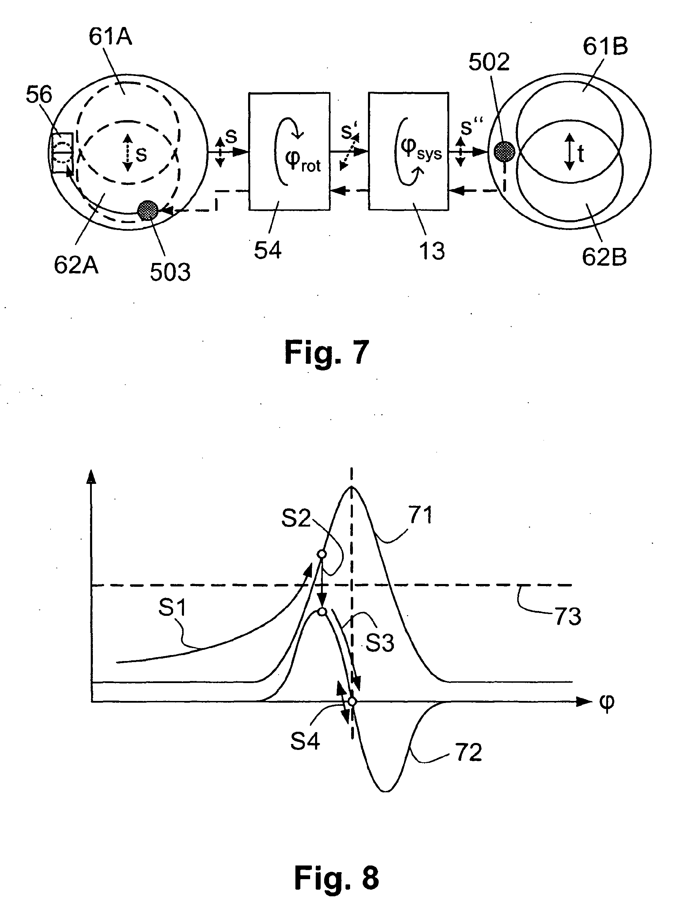

Der Laserstrahl 10 mit den vom Scanner 52 abgelenkten Lichtpulsen trifft auf das Rotationselement 54, beispielsweise ein K-Spiegel, welcher durch einen Rotationstreiber um eine optische Achse drehbar ist. Das Rotationselement 54 ermöglicht die Abtastebene, in welcher die in die Abtastrichtung s abgelenkten Lichtpulse verlaufen, um die optische Achse q zu drehen, so dass eine durch das optische Übertragungssystem des Gelenkarms 13 verursachte Verdrehung der Abtastrichtung kompensiert werden kann. Das Rotationselement 54 verdreht somit die ursprüngliche Orientierung der Abtastrichtung s in eine kompensierte Abtastrichtung s', welche durch das optische Übertragungssystem des Gelenkarms 13 in die resultierende Abtastrichtung s" verdreht wird, die der ursprünglichen Orientierung der Abtastrichtung s entspricht. Das für die Kompensation der Abtastrichtung erforderliche Ausmass der Verdrehung durch das Rotationselement 54 wird auf Grund des Referenzlichtstrahls 501 bestimmt, der vom Sender 50 aus der Senderebene B über das optische Übertragungssystem des Gelenkarms 13 an die Basisstation 11 übermittelt wird. Die Basisstation 11 umfasst ein Lichtsensormodul 56 zum Empfangen des Referenzlichtstrahls 501 in der Empfängerebene A. Das Regelmodul 55 steuert das Rotationselement 54 basierend auf dem empfangenen Referenzlichtstrahl 501, wie nachfolgend mit Bezug zu den Figuren 7 und 8 beschrieben wird.

In der Figur 7 bezeichnet das Bezugszeichen B die Senderebene, in welcher die durch den Sender 50 erzeugte Lichtmarkierung 502 sowie Querschnitte 61 B, 62B von Lichtpulsen sichtbar sind. Die Sollausrichtung der schnellen Scanbewegung ist mit t bezeichnet. Das Bezugszeichen A bezeichnet die Empfängerebene, in welcher die durch den Sender 50 erzeugte empfangene Lichtmarkierung 503 sowie Querschnitte 61A, 62A von Lichtpulsen in der Sollausrichtung sichtbar sind. In der Empfängerebene A ist zudem das Lichtsensormodul 56 angeordnet, das über zwei Lichtsensorelemente verfügt. Wie aus der Figur 7 ersichtlich ist, wird die ursprüngliche Abtastrichtung s durch das Rotationselement 54 um den Rotationswinkel ϕᵣₒₜ verdreht. Die vom Rotationselement 54 verdrehte Abtastrichtung s' wird durch das optische Übertragungssystem des Gelenkarms 13 um den Winkel ϕ_{sys} verdreht, so dass bei optimaler Regelung des Rotationselements 54 die Summe des Rotationswinkels ϕᵣₒₜ und des Winkels ϕ_{sys} Null betragen und die vom Gelenkarm 13 ausgehende Abtastrichtung s" somit der ursprünglichen Abtastrichtung s entspricht.

Wie in der Figur 8 dargestellt ist, bildet das Regelmodul 55 aus den von den beiden Lichtsensorelementen des Lichtsensormoduls 56 gemessenen Signalwerten ein Summensignal 71 und ein Differenzsignal 72. In einem ersten Schritt S1 führt das Regelmodul 55 einen Suchlauf durch, indem es den Rotationswinkel ϕᵣₒₜ so lange erhöht, bis die empfangene Lichtmarkierung 503 so in den Bereich des Lichtsensormoduls 56 verdreht wird, dass das Summensignal 71 einen definierten Schwellwert 73 überschreitet. Danach schaltet das Regelmodul 55 im Schritt S2 in einen Regelmodus um und regelt im Schritt S3 den Rotationswinkel ϕᵣₒₜ so lange, bis das Differenzsignal 72 Null beträgt. Beträgt das Differenzsignal 72 im Regelmodus Null, stimmt die Position der empfangenen Lichtmarkierung 503 mit der Position des Lichtsensormoduls 56 überein und folglich entspricht die vom Gelenkarm 13 ausgehende Abtastrichtung s" der schnellen Scanbewegung der Sollausrichtung t. Im Schritt S4 erfolgt eine laufende Feinregelung zur Kompensation von Verdrehungen der Abtastrichtung, die durch Bewegungen des Gelenkarms 13 verursacht werden.

In einer alternativen Ausführungsvariante umfasst die ophthalmologische Vorrichtung 1 anstelle des Senders 50 und des Lichtsensormoduls 56 Rotationssensoren, die jeweils in den drehbaren Armelementen des Gelenkarms 13 angebracht sind. Zur Rückmeldung des Verdrehungsgrads und der Verdrehungsrichtung des betreffenden Armelements um seine Längsachse sind die Rotationssensoren mit dem Regelmodul 55 verbunden. In dieser Ausführungsvariante bestimmt das Regelmodul 55 den Rotationswinkel ϕᵣₒₜ für das Rotationselement 54 basierend auf den Rückmeldungen der einzelnen Armelemente.

Das Steuermodul 15 und das Elektronikmodul 38 sind vorzugsweise als programmierte Softwaremodule zur Steuerung eines oder mehrerer Prozessoren der Basisstation 11 implementiert. Der Fachmann wird verstehen, dass das Steuermodul 15 und das Elektronikmodul 38 auch teilweise oder vollständig hardwaremässig ausgeführt werden können. Das Steuermodul 15 und das Elektronikmodul 38 sind eingerichtet, die Bewegungstreiber 57 so zu steuern, dass der Lichtprojektor 58, wie in der Figur 5 dargestellt, entlang einem mäandrierenden Abtastmuster 23 bewegt wird. Das Abtastmuster 23 des Lichtprojektors 58 wird durch Translationen entlang Abtastzeilen i, i+1 in der Abtastrichtung y respektive y' und durch Translationen für den Zeilenvorschub v (Zeilenabstand) in der Vorschubrichtung x gebildet. Durch die Überlagerung des Abtastmusters 26 der schnellen Scanbewegung des durch die Lichtpulse gebildeten Laserstrahls 10 auf das mäandrierende Abtastmuster 23 der mechanischen Bewegung des Lichtprojektors 58 wird die Schnittfläche 21 durch den Fokus F des projizierten Lichtstrahls flächendeckend (lückenlos) bearbeitet. Beispielsweise kann im applanierten Bereich 24 des Auges 2 ein Gewebelappen (ein so genannter Flap) vom verbleibenden Augengewebe 22 getrennt werden, der in einem Restbereich 25 (ein so genannter Hinge) mit dem Auge 2 verbunden bleibt. Das Schneiden eines solchen Flaps ist die bevorzugte Anwendung, die vorgeschlagene Vorrichtung 1 lässt sich jedoch auch für andere chirurgische Eingriffe verwenden.

Wie in der Figur 6 dargestellt ist, wird die Ablenkgeschwindigkeit des Scanners 52 vorzugsweise so bestimmt, dass sich nacheinander folgende Lichtpulse P teilweise überlagern. Die Geschwindigkeit der mechanischen Translation in der Abtastrichtung y, y' und in der Vorschubrichtung x (Slowscan-Bewegung) ist um ein Vielfaches langsamer als die Geschwindigkeit der durch die Ablenkung der Lichtpulse in der Abtastrichtung s verursachten Bewegung der Lichtpulse (Fastscan-Bewegung). Die Geschwindigkeit der mechanischen Translation in der Abtastrichtung y, y' wird vorzugsweise so bestimmt, dass sich die Lichtpulse P von nacheinander folgenden Zeilen z der schnellen Scanbewegung teilweise überlagern. Der Zeilenvorschub v des mechanischen Abtastmusters 23 wird vorzugsweise so bestimmt, dass sich das Abtastmuster 26 der schnellen Scanbewegung von einer Abtastzeile i mit dem Abtastmuster 26 der schnellen Scanbewegung der nachfolgenden Abtastzeile i+1 teilweise überlagert. Zur Erzeugung präziser Schnitte bei kurzen Schnittzeiten kommen Laserquellen mit Pulsfolgefrequenzen im Bereich zwischen 100 kHz und 100 MHz zum Einsatz. Der Durchmesser eines Lichtpulses beträgt beispielsweise 1-5 µm, die Rotations- oder Ablenkgeschwindigkeit des Scanners 52 beträgt beispielsweise 100-1000Hz, was einer Ablenkungsgeschwindigkeit der Lichtpulse in der Grössenordnung 1-10 m/s entspricht, die Geschwindigkeit der mechanischen Translation in der Abtastrichtung y, y' beträgt beispielsweise 1-10 mm/s und der Zeilenvorschub v des mechanischen Abtastmusters 23 beträgt beispielsweise 0.5-1 mm . Der in der Figur 6 angedeutete benutzte Scanbereich N wird beispielsweise durch Blenden oder mechanische oder elektronische Shutter bestimmt, die so angeordnet sind, dass die ungenutzten Scanbereiche U beispielsweise vor der dem Rotationselement 54 ausgeblendet werden.

Abschliessend soll festgehalten werden, dass der Fachmann verstehen wird, dass die vorgeschlagene Vorrichtung 1 nicht auf die beschriebenen orthogonalen Abtastraster beschränkt ist, sondern, dass die Vorrichtung 1 auch andere Abtastraster ermöglicht, beispielsweise spiral- oder kreisförmige Abtastmuster.

Schliesslich soll noch angeführt werden, dass die vorgeschlagene Vorrichtung 1 in einer Ausführungsvariante in ein Eximerlasersystem integriert ist, beispielsweise ist die Basisstation 11 in die Liege des Eximerlasersystems integriert.

## Patentansprüche

1. Ophthalmologische Vorrichtung (1), umfassend:
eine Basisstation (11) mit einer Lichtquelle (51) zur Erzeugung von Lichtpulsen, und
einen auf ein Auge (2) aufsetzbaren Applikationskopf (3)
mit einem Lichtprojektor (58) zur fokussierten Projektion der Lichtpulse für eine punktuelle Auflösung von Augengewebe (22), und
mit Bewegungstreibern (57) zur Bewegung des Lichtprojektors (58) in einer Vorschubrichtung (x) und in einer ersten Abtastrichtung (y, y'),
**gekennzeichnet durch,**
in der Basisstation (11) angeordnete Strahlablenkungsmittel zur Ablenkung der Lichtpulse in einer zweiten Abtastrichtung (s), und
ein optisches Übertragungssystem zur Übertragung abgelenkter Lichtpulse von der Basisstation (11) zum Applikationskopf (3) und zur Überlagerung der in der zweiten Abtastrichtung (s) abgelenkten Lichtpulse auf die Bewegung des Lichtprojektors (58) in der ersten Abtastrichtung (y, y').

2. Vorrichtung (1) nach Anspruch 1, **dadurch gekennzeichnet, dass** an der Basisstation (11) ein Gelenkarm (13) angebracht ist, welcher mit einem von der Basisstation (11) abgewandten Ende mit dem Applikationskopf (3) befestigbar ist, und dass das optische Übertragungssystem im Innern des Gelenkarms (13) angeordnet ist.

3. Vorrichtung (1) nach Anspruch 2, **dadurch gekennzeichnet, dass** der Gelenkarm (13) mehrere Armelemente (130, 131, 132, 133) umfasst, wobei jeweils zwei der Armelemente (130, 131, 132, 133) mittels eines Gelenkmoduls (134, 135, 136) so verbunden sind, dass sie in parallelen Ebenen zueinander verdrehbar sind, wobei mindestens eines der Armelemente (130, 131, 132, 133) um seine Längsachse drehbar ist, und wobei zur Übertragung der abgelenkten Lichtpulse von einem Armelement (130, 131, 132, 133) zum anderen Armelement (130, 131, 132, 133) in den Gelenkmodulen (134, 135, 136) Umlenkspiegel (1341, 1342, 1351, 1352, 1361, 1362) angebracht sind.

4. Vorrichtung (1) nach Anspruch 3, **dadurch gekennzeichnet, dass** an mindestens einem beweglichen Armelement (130, 131, 132, 133) des Gelenkarms (13) Mittel zur Gewichtskompensation angebracht sind, um den Applikationskopf (3) in einem Gleichgewicht zu halten.

5. Vorrichtung (1) nach einem der Ansprüche 1 bis 4, **dadurch gekennzeichnet, dass** das optische Übertragungssystem ein Rotationselement (54) umfasst zum Drehen einer durch die abgelenkten Lichtpulse definerten Abtastebene um eine optische Übertragungsachse (q).

6. Vorrichtung (1) nach Anspruch 5, **dadurch gekennzeichnet, dass** das Rotationselement (54) in der Basisstation (11) angeordnet ist.

7. Vorrichtung (1) nach einem der Ansprüche 5 oder 6, **dadurch gekennzeichnet, dass** der Applikationskopf (3) einen Sender (50) zum Aussenden einer Lichtmarkierung zur Bestimmen einer Sollausrichtung der zweiten Abtastrichtung umfasst, dass die Lichtmarkierung so angeordnet ist, dass sie über das optische Übertragungssystem zu der Basisstation (11) übertragbar ist, dass die Basisstation (11) ein Lichtsensormodul (56) umfasst, dass die Basisstation (11) ein mit dem Lichtsensormodul (56) verbundenes Regelmodul (55) umfasst, welches eingerichtet ist das Rotationselement (54) so zu steuern, dass die durch die abgelenkten Lichtpulse definierte Abtastebene so lange um die optische Übertragungsachse (q) gedreht wird, bis die Sollausrichtung der zweiten Abtastrichtung erreicht worden ist.

8. Vorrichtung (1) nach Anspruch 7, **dadurch gekennzeichnet, dass** das Lichtsensormodul (56) zwei Lichtsensoren umfasst zum Detektieren der über das optische Übertragungssystem zu der Basisstation (11) übertragenen Lichtmarkierung, und dass das Regelmodul (55) eingerichtet ist das Rotationselement (54) auf Grund einer Summe und/oder einer Differenz von durch die Lichtsensoren bestimmten Lichtstärken der detektierten Lichtmarkierung zu steuern.

9. Vorrichtung (1) nach einem der Ansprüche 5 oder 6, **dadurch gekennzeichnet, dass** der Applikationskopf (3) mittels eines mehrere um ihre Längsachse verdrehbare Armelemente (130, 131, 132, 133) umfassenden Gelenkarms (13) an der Basisstation (11) angebracht ist, dass mindestens gewisse der Armelemente (130, 131, 132, 133) mit einem Rotationssensor versehen sind, welche Rotationssensoren zur Rückmeldung eines Verdrehungsgrads des betreffenden Armelements (130, 131, 132, 133) mit einem Regelmodul (55) verbunden sind, welches Regelmodul (55) eingerichtet ist, das Rotationselement (54) so zu steuern, dass die durch die abgelenkten Lichtpulse definierte Abtastebene so lange um die optische Übertragungsachse (q) gedreht wird, bis eine Sollausrichtung der zweiten Abtastrichtung erreicht worden ist.

10. Vorrichtung (1) nach einem der Ansprüche 1 bis 9, **dadurch gekennzeichnet, dass** die Basisstation (11) ein Steuermodul (15) umfasst, welches eingerichtet ist die Bewegungstreiber (57) so zu steuern, dass der Lichtprojektor (58) äquidistant eine Arbeitsfläche in Zeilen (i, i+1), die in der ersten Abtastrichtung (y, y') verlaufen, mit einem definierten Zeilenvorschub (v) in der Vorschubrichtung abfährt, dass das optische Übertragungssystem und der Lichtprojektor (58) optisch so gekoppelt sind, dass die in der zweiten Abtastrichtung (s) abgelenkten Lichtpulse den Zeilen (i, i+1) überlagert werden, so dass die punktuelle Auflösung von Augengewebe (22) in der Arbeitsfläche zusammenhängend erfolgt.

11. Vorrichtung (1) nach einem der Ansprüche 1 bis 10, **dadurch gekennzeichnet, dass** die Lichtquelle (51) und der Lichtprojektor (58) eingerichtet sind für die punktuelle Auflösung von Augengewebe (22) an einem Brennpunkt (F) im Innern des Auges (2), dass die Basisstation (11) ein Steuermodul (15) umfasst, welches eingerichtet ist, die Bewegungstreiber (57) so zu steuern, dass durch die Lichtpulse, die in der zweiten Abtastrichtung (s) abgelenkt und der Bewegung des Lichtprojektors (58) in der ersten Abtastrichtung (y, y') überlagert sind, ein Gewebelappen, der in einem Restbereich mit dem Auge (2) verbunden bleibt, vom verbleibenden Augengewebe (22) getrennt wird.

12. Vorrichtung (1) nach einem der Ansprüche 1 bis 11, **dadurch gekennzeichnet, dass** die Bewegung des Lichtprojektors (58) im Vergleich zu einer durch die Ablenkung der Lichtpulse in der zweiten Abtastrichtung (s) verursachten Bewegung der Lichtpulse um ein Vielfaches langsamer ist.

13. Vorrichtung (1) nach einem der Ansprüche 1 bis 12, **dadurch gekennzeichnet, dass** die in der Basisstation (11) angeordneten, Strahlablenkungsmittel einen drehbar gelagerten Spiegel (521) mit Antriebsmitteln (522) umfassen, insbesondere einen Polygonspiegel mit mehreren Facetten, dass das Rotationselement (54) als K-Spiegel ausgestaltet ist, dass der Applikationskopf (3) einen auf das Auge (2) aufsetzbaren, mindestens stellenweise lichtdurchlässigen Kontaktkörper (31) umfasst, welcher so ausgebildet und angeordnet ist, dass er einen im aufgesetzten Zustand kontaktierten Bereich des Auges (2) äquidistant zu einer Arbeitsfläche setzt, dass der Applikationskopf (3) Befestigungsmittel aufweist zum Fixieren des Applikationskopfs (3) durch Unterdruck am Auge (2), und dass die Lichtquelle (51) einen Femtosekundenlaser (511) umfasst.

14. Vorrichtung (1) nach einem der Ansprüche 1 bis 13, **dadurch gekennzeichnet, dass** die Basisstation (11) in ein Eximerlasersystem integriert ist.

## Claims

1. Ophthalmological apparatus (1), comprising:
a base station (11) having a light source (51) for generating light pulses, and
an application head (3) which can be mounted on an eye (2)
having a light projector (58) for the focussed projection of the light pulses for punctiform breakdown of eye tissue (22), and
having movement drivers (57) for moving the light projector (58) in a feed direction (x) and in a first scanning direction (y, y'),
**characterized by**
beam deflecting means, arranged in the base station (11), for deflecting the light pulses in a second scanning direction (s), and
an optical transmission system for transmitting deflected light pulses from the base station (11) to the application head (3), and for superimposing the light pulses deflected in the second scanning direction (s) onto the movement of the light projector (58) in the first scanning direction (y, y').

2. Apparatus (1) according to Claim 1, **characterized in that** fitted on the base station (11) is an articulated arm (13) which can be fastened to the application head (3) with an end diverted from the base station (11), and **in that** the optical transmission system is arranged in the interior of the articulated arm (13).

3. Apparatus (1) according to Claim 2, **characterized in that** the articulated arm (13) comprises a number of arm elements (130, 131, 132, 133), in each case two of the arm elements (130, 131, 132, 133) being connected by means of an articulated module (134, 135, 136) such that they can be rotated in parallel planes relative to one another, at least one of the arm elements (130, 131, 132, 133) being rotatable about its longitudinal axis, and deflecting mirrors (1341, 1342, 1351, 1352, 1361, 1362) being fitted in the articulated modules (134, 135, 136) in order to transmit the deflected light pulses from one arm element (130, 131, 132, 133) to the other arm element (130, 131, 132, 133).

4. Apparatus (1) according to Claim 3, **characterized in that** means for weight compensation are fitted on at least one moveable arm element (130, 131, 132, 133) of the articulated arm (13) in order to keep the application head (3) in equilibrium.

5. Apparatus (1) according to one of Claims 1 to 4, **characterized in that** the optical transmission system comprises a rotation element (54) for rotating a scanning plane defined by the deflected light pulses about an optical transmission axis (q).

6. Apparatus (1) according to Claim 5, **characterized in that** the rotation element (54) is arranged in the base station (11).

7. Apparatus (1) according to one of Claims 5 or 6, **characterized in that** the application head (3) comprises a transmitter (50) for emitting a light mark for determining a desired alignment of the second scanning direction, **in that** the light mark is arranged such that it can be transmitted to the base station (11) via the optical transmission system, **in that** the base station (11) comprises a light sensor module (56), and **in that** the base station (11) comprises a regulation module (55) which is connected to the light sensor module (56) and is set up to control the rotation element (54) so that the scanning plane defined by the deflected light pulses is rotated about the optical transmission axis (q) until the desired alignment of the second scanning direction has been reached.

8. Apparatus (1) according to Claim 7, **characterized in that** the light sensor module (56) comprises two light sensors for detecting the light mark transmitted via the optical transmission system to the base station (11), and **in that** the regulation module (55) is set up to control the rotation element (54) on the basis of a sum and/or a difference of luminosities, determined by the light sensors, of the detected light mark.

9. Apparatus (1) according to either of Claims 5 or 6, **characterized in that** the application head (3) is fitted on the base station (11) by means of an articulated arm (13) comprising a number of arm elements (130, 131, 132, 133) which can be rotated about their longitudinal axis, and **in that** at least certain ones of the arm elements (130, 131, 132, 133) are provided with a rotation sensor, which rotation sensors are connected to a regulation module (55) for the purpose of feeding back a degree of rotation of the relevant arm element (130, 131, 132, 133), which regulation module (55) is set up to control the rotation element (54) so that the scanning plane defined by the deflected light pulses is rotated about the optical transmission axis (q) until a desired alignment of the second scanning direction has been reached.

10. Apparatus (1) according to one of Claims 1 to 9, **characterized in that** the base station (11) comprises a control module (15) which is set up to control the movement drivers (57) so that the light projector (58) traverses a working surface equidistantly in lines (i, i+1), which run in the first scanning direction (y, y'), with a defined line feed (v) in the feed direction, and **in that** the optical transmission system and the light projector (58) are optically coupled such that the light pulses deflected in the second scanning direction (s) are superimposed on the lines (i, i+1) such that the punctiform breakdown of eye tissue (22) in the working surface is performed coherently.

11. Apparatus (1) according to one of Claims 1 to 10, **characterized in that** the light source (51) and the light projector (58) are set up for the punctiform breakdown of eye tissue (22) at a focal point (F) in the interior of the eye (2), and **in that** the base station (11) comprises a control module (15) which is set up to control the movement drivers (57) so that the light pulses which are deflected in the second scanning direction (s) and are superimposed on the movement of the light projector (58) in the first scanning direction (y, y') separate from the remaining eye tissue (22) a tissue flap which remains connected to the eye (2) in a residual area.

12. Apparatus (1) according to one of Claims 1 to 11, **characterized in that** the movement of the light projector (58) is slower by a multiple by comparison with a movement of the light pulses which is caused by the deflection of the light pulses in the second scanning direction (s).

13. Apparatus (1) according to one of Claims 1 to 12, **characterized in that** the beam deflecting means arranged in the base station (11) comprise a rotatably supported mirror (521) having drive means (522), in particular a polygonal mirror having a number of facets, **in that** the rotation element (54) is configured as a K-mirror, **in that** the application head (3) comprises a contact body (31) which can be mounted on the eye (2), is transparent at least in parts and is configured and arranged so that it sets a region of the eye (2) whose contact is made in the mounted state in a fashion equidistant from a working surface, **in that** the application head (3) has fastening means for fixing the application head (3) at the eye (2) by low pressure, and **in that** the light source (51) comprises a femtosecond laser (511).

14. Apparatus (1) according to one of Claims 1 to 13, **characterized in that** the base station (11) is integrated in an eximer laser system.

## Revendications

1. Dispositif ophtalmologique (1), comprenant :
un poste de base (11) avec une source de lumière (51) pour produire des impulsions de lumière, et
une tête d'application (3) pouvant être posée sur un oeil (2),
avec un projecteur de lumière (58) pour la projection focalisée de l'impulsion de lumière pour une désintégration ponctuelle du tissu oculaire (22), et
avec des dispositifs d'entraînement en mouvement (57) pour le déplacement du projecteur de lumière (58) dans une direction d'avance (x) et dans une première direction de balayage (y, y'),
**caractérisé par**
des moyens de déviation de faisceau disposés dans le poste de base pour dévier l'impulsion de lumière dans une deuxième direction de balayage (s), et
un système de transfert optique pour transférer des impulsions de lumière déviées du poste de base (11) vers la tête d'application (3) et pour superposer les impulsions de lumière déviées dans la deuxième direction de balayage (s) au déplacement du projecteur de lumière (58) dans la première direction de balayage (y, y').

2. Dispositif (1) selon la revendication 1, **caractérisé en ce que** l'on monte sur le poste de base (11) un bras articulé (13) qui peut être fixé à la tête d'application (3) par une extrémité opposée au poste de base (11), et **en ce que** le système de transfert optique est disposé à l'intérieur du bras articulé (13).

3. Dispositif (1) selon la revendication 2, **caractérisé en ce que** le bras articulé (13) présente plusieurs éléments de bras (130, 131, 132, 133), à chaque fois deux des éléments de bras (130, 131, 132, 133) étant connectés au moyen d'un module articulé (134, 135, 136) de telle sorte qu'ils puissent tourner l'un par rapport à l'autre dans des plans parallèles, au moins l'un des éléments de bras (130, 131, 132, 133) pouvant tourner autour de son axe longitudinal et pour le transfert des impulsions de lumière déviées d'un élément de bras (130, 131, 132, 133) à un autre élément de bras (130, 131, 132, 133), des miroirs de déviation (1341, 1342, 1351, 1352, 1361, 1362) étant montés dans les modules articulés (134, 135, 136).

4. Dispositif (1) selon la revendication 3, **caractérisé en ce que** des moyens pour la compensation du poids sont montés sur au moins un élément de bras (130, 131, 132, 133) mobile du bras articulé (13), afin de maintenir la tête d'application (3) en équilibre.

5. Dispositif (1) selon l'une quelconque des revendications 1 à 4, **caractérisé en ce que** le système de transfert optique comprend un élément de rotation (54) pour faire tourner un plan de balayage, défini par les impulsions de lumière déviées, autour d'un axe de transfert optique (q).

6. Dispositif (1) selon la revendication 5, **caractérisé en ce que** l'élément de rotation (54) est disposé dans le poste de base (11).

7. Dispositif (1) selon l'une quelconque des revendications 5 ou 6, **caractérisé en ce que** la tête d'application (3) comprend un émetteur (50) pour envoyer un marquage de lumière pour déterminer une orientation de consigne du deuxième dispositif de balayage, **en ce que** le marquage de lumière est disposé de telle sorte qu'il puisse être transmis par le biais du système de transfert optique au poste de base (11), **en ce que** le poste de base (11) comprend un module de capteur de lumière (56), **en ce que** le poste de base (11) comprend un module de réglage (55) connecté au module de capteur de lumière (56), qui est prévu pour commander l'élément de rotation (54) de telle sorte que le plan de balayage défini par les impulsions de lumière déviées soit tourné autour de l'axe de transfert optique (q) jusqu'à ce que l'orientation de consigne de la deuxième direction de balayage soit atteinte.

8. Dispositif (1) selon la revendication 7, **caractérisé en ce que** le module de capteur de lumière (56) comprend deux capteurs de lumière pour détecter le marquage de lumière transmis par le biais du système de transfert optique au poste de base (11), et **en ce que** le module de réglage (55) est prévu pour commander l'élément de rotation (54) sur la base d'une somme et/ou d'une différence des intensités lumineuses déterminées par les capteurs de lumière du marquage de lumière détecté.

9. Dispositif (1) selon l'une quelconque des revendications 5 ou 6, **caractérisé en ce que** la tête d'application (3) est montée sur le poste de base (11) au moyen d'un bras articulé (13) comprenant plusieurs éléments de bras (130, 131, 132, 133) pouvant tourner autour de leur axe longitudinal, **en ce qu'**au moins certains des éléments de bras (130, 131, 132, 133) sont pourvus d'un capteur de rotation, ces capteurs de rotation étant connectés à un module de réglage (55) pour communiquer en retour un degré de rotation de l'élément de bras concerné (130, 131, 132, 133), ledit module de réglage (55) étant prévu pour commander l'élément de rotation (54) de telle sorte que le plan de balayage défini par les impulsions de lumière déviées soit tourné autour de l'axe de transfert optique (q) jusqu'à ce qu'une orientation de consigne de la deuxième direction de balayage soit atteinte.

10. Dispositif (1) selon l'une quelconque des revendications 1 à 9, **caractérisé en ce que** le poste de base (11) comprend un module de commande (15) qui est prévu pour commander les dispositifs d'entraînement en mouvement (57) de telle sorte que le projecteur de lumière (58) se déplace dans la direction d'avance avec une avance de lignes définie (v) de manière équidistante à une surface de travail en lignes (i, i+1), qui s'étendent dans la première direction de balayage (y, y'), **en ce que** le système de transfert optique et le projecteur de lumière (58) sont accouplés optiquement de telle sorte que les impulsions de lumière déviées dans la deuxième direction de balayage (s) soient superposées aux lignes (i, i+1), de sorte que la désintégration ponctuelle du tissu oculaire (22) s'effectue en continu dans la surface de travail.

11. Dispositif (1) selon l'une quelconque des revendications 1 à 10, **caractérisé en ce que** la source de lumière (51) et le projecteur de lumière (58) sont prévus pour la désintégration ponctuelle du tissu oculaire (22) en un foyer (F) à l'intérieur de l'oeil (2), **en ce que** le poste de base (11) comprend un module de commande (15) qui est prévu pour commander les dispositifs d'entraînement en mouvement (57) de telle sorte qu'un lambeau tissulaire, qui reste rattaché à l'oeil (2) dans une région résiduelle, soit séparé du reste du tissu oculaire (22) par les impulsions de lumière qui sont déviées dans la deuxième direction de balayage (s) et qui sont superposées au mouvement du projecteur de lumière (58) dans la première direction de balayage (y, y').

12. Dispositif (1) selon l'une quelconque des revendications 1 à 11, **caractérisé en ce que** le déplacement du projecteur de lumière (58) est plus lent d'un multiple par comparaison avec un mouvement des impulsions de lumière provoqué par la déviation des impulsions de lumière dans la deuxième direction de balayage (s).

13. Dispositif (1) selon l'une quelconque des revendications 1 à 12, **caractérisé en ce que** les moyens de déviation de faisceau disposés dans le poste de base (11) comprennent un miroir (521) monté à rotation avec des moyens d'entraînement (522), en particulier un miroir polygonal avec plusieurs facettes, **en ce que** l'élément de rotation (54) est réalisé sous forme de miroir en K, **en ce que** la tête d'application (3) présente un corps de contact (31) pouvant être placé sur l'oeil (2), au moins en partie perméable à la lumière, qui est réalisé et disposé de telle sorte qu'il place une région de l'oeil (2), en contact avec lui dans l'état posé, de manière équidistante à une surface de travail, **en ce que** la tête d'application (3) présente des moyens de fixation pour la fixation de la tête d'application (3) par dépression sur l'oeil (2), et **en ce que** la source de lumière (51) comprend un laser femtoseconde (511).

14. Dispositif (1) selon l'une quelconque des revendications 1 à 13, **caractérisé en ce que** le poste de base (11) est intégré dans un système de laser excimère.
